# EUROPEAN PATENT SPECIFICATION

(11) **EP 4 029 557 B1**
(45) Date of publication and mention of the grant of the patent: **04.10.2023**
(21) Application number: 20926137.9
(22) Date of filing: 01.06.2020
(51) Int. Cl.: A61M 31/00, A61M 35/00, A61M 25/00

(54) **ASSISTING DEVICE FOR LIQUID DRUG PERMEATION APPARATUS AND LIQUID DRUG PERMEATION SYSTEM**
HILFSVORRICHTUNG FÜR EINE VORRICHTUNG ZUR PERMEATION VON FLÜSSIGEN MEDIKAMENTEN UND SYSTEM ZUR PERMEATION VON FLÜSSIGEN MEDIKAMENTEN
DISPOSITIF D'ASSISTANCE POUR APPAREIL DE PERMÉATION DE MÉDICAMENTS LIQUIDES ET SYSTÈME DE PERMÉATION DE MÉDICAMENTS LIQUIDES

(30) Priority: 17.03.2020 JP 2020046824
(43) Date of publication of application: 20.07.2022
(73) Proprietor: AceMedic Inc., Okayama-shi, Okayama 700-0961 (JP)
(72) Inventor: WATANABE, Masami, Okayama-shi, Okayama 700-8558 (JP); SADAHIRA, Takuya, Okayama-shi, Okayama 700-8558 (JP)
(74) Representative: Winter, Brandl - Partnerschaft mbB
(86) International application number: PCT/JP2020/021545
(87) International publication number: WO 2021/186752

(56) References cited:
- WO-A1-2007/011040
- JP-A- 2010 274 123
- JP-A- 2015 211 828
- JP-A- 2015 528 724
- US-A1- 2016 256 634

## Description

### Technical Field

The present invention relates to an assisting device for a liquid drug permeation apparatus and a liquid drug permeation system to be used in a therapy involving causing a liquid drug to permeate to an affected area, such as cancer.

### Background Art

A therapeutic method for causing a liquid drug to permeate to an affected area, such as cancer, has hitherto been known. In particular, in recent years, the performance of an endoscope has been improved, and hence a procedure for causing an anticancer drug to permeate to cancer in a body through use of the endoscope has been investigated.

In order to efficiently perform such permeation of a liquid drug through use of the endoscope, the inventors of the present invention have developed a permeation apparatus configured to cause a liquid drug to permeate to an affected area by causing a water-absorbing material impregnated with the liquid drug to protrude from a forceps port at a distal end of the endoscope and bringing the water-absorbing material into abutment against the affected area under visual recognition with the endoscope (see, for example, Patent Literature 1).

In the permeation apparatus of the above-mentioned patent literature, the water-absorbing material is mounted on a distal end of an inner tube configured to supply the liquid drug, and the inner tube is inserted in an inside of an outer tube configured to aspirate a part of the liquid drug supplied to the water-absorbing material.

Here, the inner tube communicates to and is coupled to a supply syringe configured to supply the liquid drug. With this, the liquid drug can be supplied from the supply syringe through the inner tube at a constant flow rate. The outer tube communicates to and is coupled to an aspiration tool, such as an aspiration syringe. With this, when an aspirator is operated in an aspiration direction, the inside of the outer tube is brought into a negative pressure, and a part of the liquid drug of the water-absorbing material can be aspirated into the outer tube. By supplying the liquid drug to the water-absorbing material through the inner tube or by recovering the liquid drug from a distal end of the outer tube, the water-absorbing material can be always maintained in a wet state.

The outer tube having the inner tube inserted therein is inserted in a forceps insertion port of the endoscope, and is caused to pass through a forceps conduit of the endoscope and extend from a forceps port. In this case, the liquid drug can be applied to and caused to permeate to the predetermined affected area with the water-absorbing material while the water-absorbing material is advanced by advancing the outer tube through which the water-absorbing material protrudes from the forceps port with respect to the forceps insertion port or conversely while the water-absorbing material is retracted by retracting the outer tube with respect to the forceps insertion port, or while the distal end of the endoscope is shaken horizontally and vertically or while the distal end is bent.

### Citation List

### Patent Literature

PTL 1: JP 2015 - 211 828 A

### Summary of Invention

### Technical Problem

In the above-mentioned permeation apparatus, there is no problem under a state in which the supply of the liquid drug by the inner tube and the aspiration of the liquid drug by the outer tube are balanced. However, the balance between the supply and the aspiration of the liquid drug may be lost depending on the state of the affected area. For example, when the aspiration of the liquid drug by the outer tube is insufficient, an excess liquid drug is accumulated in the affected area, and there is a risk in that liquid dripping of the liquid drug may occur in the water-absorbing material.

In particular, when the liquid drug, such as an anticancer drug, is used, the liquid drug itself may be a dangerous drug in many cases. Therefore, it is required to pay attention so that the liquid drug does not adhere also to a normal tissue that is not a cancer tissue, and hence it is required to prevent the liquid dripping from occurring. The term "liquid dripping" as used herein refers to a state in which the overflow liquid drug that cannot be held by the water-absorbing material drips due to the contact with an affected area surface or the like or due to the gravity or the like and flows out to an unexpected region. The accumulated state of the liquid drug immediately before the occurrence of the liquid dripping is referred to as "liquid accumulation".

When the liquid accumulation occurs in the water-absorbing material, it is theoretically conceivable that the liquid accumulation can be eliminated by suspending the supply of the liquid drug from the inner tube or increasing the aspiration of the liquid drug by the outer tube. However, in actuality, it may be too late to eliminate the liquid accumulation due to the presence of time lag, and it is required to accurately eliminate the liquid accumulation in a shorter period of time.

### Solution to Problem

This object is solved by the subject matter of independent claim 1. Further aspects are disclosed in the subclaims. In order to solve the above-mentioned problem, the inventors of the present application have developed an assisting device, which is configured to assist position adjustment of the above-mentioned liquid drug permeation apparatus under an endoscope, and which includes a fixture configured to fix the inner tube of the liquid drug permeation apparatus to the assisting device. The liquid accumulation is prevented by fixing the inner tube of the liquid drug permeation apparatus to the assisting device with the fixture and advancing the outer tube with respect to the inner tube of the liquid drug permeation apparatus so as to allow the water-absorbing material to be pulled into the outer tube. Thus, the present invention has been completed.

According to one embodiment of the present invention, there is provided a liquid drug permeation system for causing permeation of a liquid drug under an endoscope, including: a liquid drug permeation apparatus; and an assisting device configured to assist position adjustment of the liquid drug permeation apparatus under the endoscope, wherein the liquid drug permeation apparatus includes an outer tube and an inner tube inserted in the outer tube, the inner tube has a water-absorbing material for absorbing a liquid drug mounted on a distal end, and a part of the liquid drug supplied through the inner tube is aspirated through the outer tube, wherein the assisting device includes a connector portion to be mounted on the endoscope and a fixture configured to fix the inner tube to the assisting device, and wherein, when the connector portion of the assisting device is mounted on the endoscope and the inner tube is fixed to the assisting device with the fixture, the outer tube is allowed to selectively advance and retract with respect to the inner tube, and the water-absorbing material mounted on the distal end of the inner tube is allowed to be pulled into the outer tube by advancing the outer tube with respect the inner tube.

There is described a method, not being part of the invention, of operating a liquid drug permeation system, including: causing a distal end of the water-absorbing material impregnated with the liquid drug to protrude from the distal end forceps port of the endoscope, releasing the liquid drug from the water-absorbing material under a state in which the distal end of the outer tube is located at a first distance from the distal end of the water-absorbing material; and advancing the outer tube with respect to the inner tube to retract the water-absorbing material further into the outer tube to thereby set a position of the outer tube so that a distance between the distal end of the outer tube and the distal end of the water-absorbing material reaches a second distance shorter than the first distance (not covered by the present claims).

According to still another embodiment of the present invention, there is provided an assisting device configured to assist position adjustment of the liquid drug permeation apparatus under the endoscope, wherein the liquid drug permeation apparatus includes an outer tube and an inner tube inserted in the outer tube, the inner tube has a water-absorbing material for absorbing a liquid drug mounted on a distal end, and a part of the liquid drug supplied through the inner tube is aspirated through the outer tube, wherein the assisting device includes a connector portion to be mounted on the endoscope and a fixture configured to fix the inner tube to the assisting device, and wherein, when the connector portion of the assisting device is mounted on the endoscope and the inner tube is fixed to the assisting device with the fixture, the outer tube is allowed to selectively advance and retract with respect to the inner tube, and the water-absorbing material is allowed to be pulled into the outer tube by advancing the outer tube with respect the inner tube.

### Advantageous Effects of Invention

According to the present invention, the outer tube is allowed to selectively advance and retract with respect to the inner tube. Therefore, when liquid accumulation leading to liquid dripping occurs, the liquid accumulation of the liquid drug can be accurately eliminated in a shorter period of time by advancing the outer tube with respect to the inner tube to retract a part of the water-absorbing material into the outer tube.

### Brief Description of Drawings

FIG. 1A is an explanatory view of a liquid drug permeation system according to a first embodiment of the present invention.
FIG. 1B is an enlarged view of a portion surrounded by the alternate long and short dash line 1B of FIG. 1A.
FIG. 2 is a schematic diagram for illustrating a supply syringe connected to an inner tube and an aspiration syringe connected to an outer tube.
FIG. 3 is an explanatory view of a mode of mounting a rod including a fixture on an endoscope.
FIG. 4A is an explanatory view of an advancing operation of the outer tube of the liquid drug permeation system of FIG. 1A.
FIG. 4B is an enlarged view of a portion surrounded by the alternate long and short dash line 4B of FIG. 4A.
FIG. 4C is an explanatory view in which the outer tube is further advanced from the state of FIG. 4A.
FIG. 4D is an enlarged view of a portion surrounded by the alternate long and short dash line 4D of FIG. 4C.
FIG. 5A is an explanatory view of a use method of the liquid drug permeation system of FIG. 1A.
FIG. 5B is an explanatory view of the use method of the liquid drug permeation system of FIG. 1A.
FIG. 6 is an explanatory view of another use method of the liquid drug permeation system.
FIG. 7 is an explanatory view of another use method of the liquid drug permeation system.
FIG. 8A is an explanatory view of another use method of the liquid drug permeation system.
FIG. 8B is an explanatory view for illustrating a state in which an insertion tube is further advanced from FIG. 8A.
FIG. 9A is an explanatory view of an advancing operation of an outer tube of a liquid drug permeation system according to a second embodiment of the present invention.
FIG. 9B is an enlarged view of a portion surrounded by the alternate long and short dash line 9B of FIG. 9A.
FIG. 9C is an explanatory view in which the outer tube is further advanced from the state of FIG. 9A.
FIG. 9D is an enlarged view of a portion surrounded by the alternate long and short dash line 9D of FIG. 9C.
FIG. 10A is a schematic front view for illustrating another embodiment of a fixture in which an inner tube is disengaged.
FIG. 10B is a schematic front view of the fixture of FIG. 10A in which the movement of the inner tube is restricted.
FIG. 11 is an explanatory view for illustrating a sensing means mounted in the vicinity of a distal end of the inner tube.
FIG. 12 is an explanatory view of a base connector of the outer tube.
FIG. 13 is a photograph for showing a periphery of an affected area when a 50% acetic acid solution is applied to the stomach wall mucosa of a beagle dog through use of the liquid drug permeation system.
FIG. 14A is a photograph of a periphery of a raised portion created artificially on the stomach mucosa of the beagle dog before the inner tube of the drug permeation apparatus is inserted and advanced to the raised portion.
FIG. 14B is a photograph of the periphery of the raised portion created artificially on the stomach mucosa of the beagle dog after the inner tube of the drug permeation apparatus is inserted and advanced to the raised portion.

### Description of Embodiments

Now, detailed description is given of embodiments of the present invention with reference to the drawings.

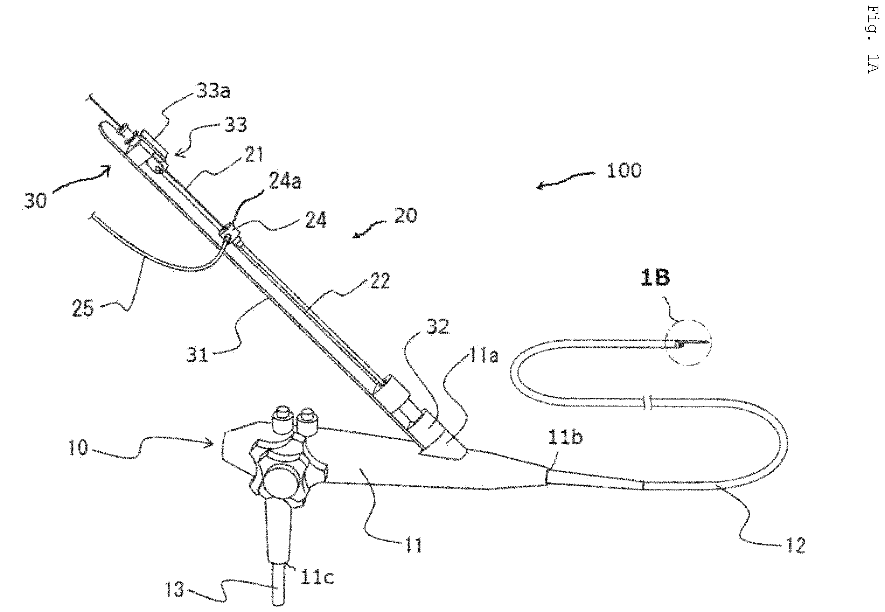

As illustrated in FIG. 1A, a liquid drug permeation system 100 according to a first embodiment of the present invention is used together with an endoscope 10, and is configured to cause permeation of a liquid drug under the endoscope 10. A combination of the liquid drug permeation system 100 according to the first embodiment and the endoscope 10 may also be regarded as a liquid drug permeation system. The liquid drug is not particularly limited, and may be any liquid drugs: for example, an anticancer drug, an antimicrobial drug, a gastrointestinal drug, a low-molecular compound, nucleic acid medicine containing DNA, RNA, or the like, an antibody drug, a gene therapeutic drug, a diagnostic drug, a drug containing a microorganism, such as a bacterium or a virus; a drug containing cytokine, chemokine, a functional protein, or an amino acid; cell medicine containing a cell, a medical drug (including medicine containing a cell) for regeneration medicine, a drug containing a radioactive isotope; and a drug containing a photosensitive substance. Specific examples of the substance include, but not limited to, ethanol, acetic acid, trichloroacetic acid, monochloroacetic acid, dimethyl sulfoxide (DMSO), glutaraldehyde, formaldehyde, phenol, silver nitride, hydrochloric acid, sodium hydroxide, DNA, RNA, an amino acid, and a protein.

The endoscope 10 includes a stick-shaped operation unit 11, an insertion tube 12 extending from a distal end forceps port 11b at a distal end of the operation unit 11, a connector cable 13 extending from an opening 11c on a side surface of the operation unit 11, and a connector portion (not shown) provided at a distal end of the connector cable 13.

The operation unit 11 includes a left-right angle knob, an up-down angle knob, air supply and water supply buttons, an aspiration button, and the like (not shown) so that various operations can be performed. A forceps insertion port 11a is formed on a side surface opposite to the opening 11c of the operation unit 11. A conduit (not shown) that communicates to the forceps insertion port 11a is arranged in the insertion tube 12. When the endoscope 10 is used under a state in which forceps are inserted therein, the forceps inserted in the forceps insertion port 11a is caused to pass through the conduit so that the forceps can protrude from a distal end of the insertion tube 12 through the inside of the insertion tube 12.

The liquid drug permeation system 100 according to the first embodiment of the present invention includes a liquid drug permeation apparatus 20 configured to cause permeation of the liquid drug under the endoscope 10 and an endoscopic permeation apparatus assisting device 30 (hereinafter sometimes simply referred to as "assisting device") configured to assist position adjustment of the liquid drug permeation apparatus under the endoscope 10.

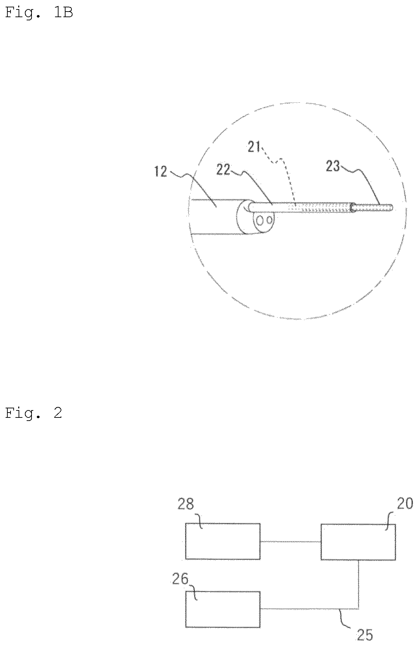

The liquid drug permeation apparatus 20 includes an outer tube 22 and an inner tube 21 inserted in the outer tube 22. A water-absorbing material 23 (FIG. 1B) for absorbing the liquid drug is mounted on a distal end of the inner tube 21. The inner tube 21 is coaxially inserted in the inside of the outer tube 22, and the dual tube is inserted in the forceps insertion port 11a of the endoscope 10 and used. A branch connector 24 that allows the inner tube 21 to be inserted therein so that the inner tube 21 can selectively advance and retract is mounted on a proximal end portion of the outer tube 22, and the inner tube 21 is inserted in a through opening of the branch connector 24 to form a double tube state of the inner tube 21 and the outer tube 22. In order to facilitate or support rotation of the outer tube 22 by an operator, the outer diameter of an annular main body 24a of the branch connector 24 is formed to be larger than the outer diameter of the outer tube 22. Therefore, the operator can easily move the outer tube 22 along a longitudinal direction by gripping the branch connector 24. In addition, the operator can easily rotate the outer tube 22 about a longitudinal direction axis by gripping and rotating the branch connector 24.

One end of a coupling tube 25 is connected to a side surface of the branch connector 24, and another end of the coupling tube 25 communicates to and is coupled to an aspiration syringe 26 (FIG. 2) forming an aspiration device. When a piston of the aspiration syringe 26 is operated in an aspiration direction, the inside of the outer tube 22 can be adjusted to a negative pressure. As operation means for operating the piston of the aspiration syringe 26 in the aspiration direction, an appropriate actuator or the like may be used. The aspiration condition can be appropriately set by the operator, such as a physician. For example, the following control is performed. Air is aspirated for 5 seconds at a speed of 1.5 mL/sec per one time through use of the aspiration syringe 26, and the aspiration is repeated intermittently and periodically at intervals of once every 30 seconds. Alternatively, air is always aspirated at a speed of 0.5 mL/sec.

A proximal end of the inner tube 21 communicates to and is coupled to a supply syringe 28 (FIG. 2) configured to supply the liquid drug, which forms a liquid drug supply device configured to supply the liquid drug, so that the liquid drug can be supplied or injected to the inner tube 21 with the supply syringe 28 at a constant flow rate. Also as operation means for operating a piston of the supply syringe 28, an appropriate actuator or the like may be used. The supply or injection condition can be appropriately set by the operator, such as a physician. For example, the following control is performed. 50 vol% acetic acid (balance is a 50 vol% indigocarmine solution) is always injected at a speed of 40 µL/min.

The inner tube 21 is inserted in the branch connector 24 to be inserted in the outer tube 22 to form a double tube as illustrated in FIG. 1A, and the distal end of the inner tube 21 is caused to protrude as compared to a distal end of the outer tube 22 as illustrated in FIG. 1B. The protruding length of the distal end of the inner tube 21 with respect to the outer tube 22 may be set to an appropriate length, but in this embodiment, the distal end of the inner tube 21 is caused to protrude by about 1 cm.

The water absorbing material 23 for absorbing the liquid drug is mounted on the distal end of the inner tube 21 protruding from the outer tube 22. When the inner tube 21 is not fixed with a fixture 33 described later, the inner tube 21 is movable relative to the outer tube 22. Therefore, the entire water-absorbing material 23 can be caused to protrude outward from the outer tube 33 by advancing the inner tube 21, and the entire water-absorbing material 23 can be positioned in the outer tube 33 by retracting the inner tube 21. The water-absorbing material 23 is brought into a wet state with the liquid drug supplied from the supply syringe 28 through the inner tube 21. It is only required that the water-absorbing material 23 have a water-absorbing property, and the water-absorbing material 23 may be made of a hydrophilic polymer, a foam capable of holding an aqueous liquid, such as a body fluid, or knitted and woven fabric or nonwoven fabric containing a water-absorbing fiber.

Examples of the hydrophilic polymer include polyvinyl alcohol (PVA), polyethylene glycol, polyvinyl pyrrolidone (PVP), methyl cellulose, ethyl cellulose, hydroxypropylmethyl cellulose, carboxymethyl cellulose, chitosan, and agarose. Examples of the foam include a melamine resin foam, a polyurethane foam, a polystyrene foam, a polyolefin foam, a phenol resin foam, a polyvinyl chloride (PVC) foam, a urea resin (UF) foam, a silicone (SI) foam, and a polyimide (PI) foam. Examples of the water-absorbing fiber include rayon, cotton, linen, and wool. In this embodiment, cotton is used for the water-absorbing material 23.

It is preferred that the water-absorbing material 23 cover the entire surface of a portion of the inner tube 21 that protrudes from the outer tube 22 and a proximal end of the water-absorbing material 23 be positioned in the outer tube 22. That is, it is preferred that the dimension of the water-absorbing material 23 in a length direction of the inner tube 21 be set to be longer than the length of the portion of the inner tube 21 that protrudes from the outer tube 22. The dimension of the water-absorbing material 23 in the length direction of the inner tube 21 is not limited, but is, for example, from 0.1 mm to 5 cm, preferably from 1 mm to 3 cm. In addition, it is preferred that the outer peripheral diameter dimension of the water-absorbing material 23 in a state of being mounted on the inner tube 21 be set to be smaller than the inner peripheral diameter dimension of the outer tube 22. With this, as described later, when the outer tube 22 is advanced with respect to the inner tube 21, there is no risk in that the water-absorbing material 23 may be detached with the advancing outer tube 22, and the outer tube 22 can be smoothly advanced over the water-absorbing material 23, with the result that the water-absorbing material 23 can be pulled into the outer tube 22.

In addition, when the proximal end (end on the outer tube 22 side) of the water-absorbing material 23 is positioned in the outer tube 22, the water-absorbing material 23 can be prevented from the detachment, and the liquid drug in the water-absorbing material 23 is also easily aspirated with the outer tube 22 brought into a negative pressure state. That is, a flow for aspirating, by the outer tube 22, the liquid drug supplied to the water absorbing material 23 by the inner tube 21 can be generated, and the liquid drug can be smoothly supplied.

The assisting device 30 includes a rod 31 serving as an elongated body. A connector portion 32 to be mounted on the endoscope 10 is provided at one end of the rod 31, and the fixture 33 configured to fix the inner tube 21 to the assisting device 30 is provided at another end of the rod 31. A female screw is formed in the connector portion 32, and a male screw is formed in the forceps insertion port 11a. Therefore, the connector portion 32 can be detachably screwed into the forceps insertion port 11a. In this embodiment, the rod portion 31 is formed into a flat plate shape, but the rod 31 may be formed into an appropriate shape.

The fixture 33 includes a rubber tube (not shown) in which the inner tube 21 is inserted, a wedge plate (not shown) configured to crush the rubber tube toward a center, and a lock lever 33a connected to the wedge plate. The lock lever 33a can be turned to a lock position and an unlock position. When the lock lever 33a is turned to the lock position, the rubber tube is crushed by the wedge plate, and the position of the inner tube 21 is fixed when the inner tube 21 is brought into a state of being held by the rubber tube. When the lock lever 33a is turned to the unlock position, the wedge plate moves in a direction of being separated from the rubber tube to plastically restore the rubber tube, with the result that the inner tube 21 can move.

The advancing operation of the outer tube 22 with respect to the inner tube 21 of the liquid drug permeation apparatus 20 is described below.

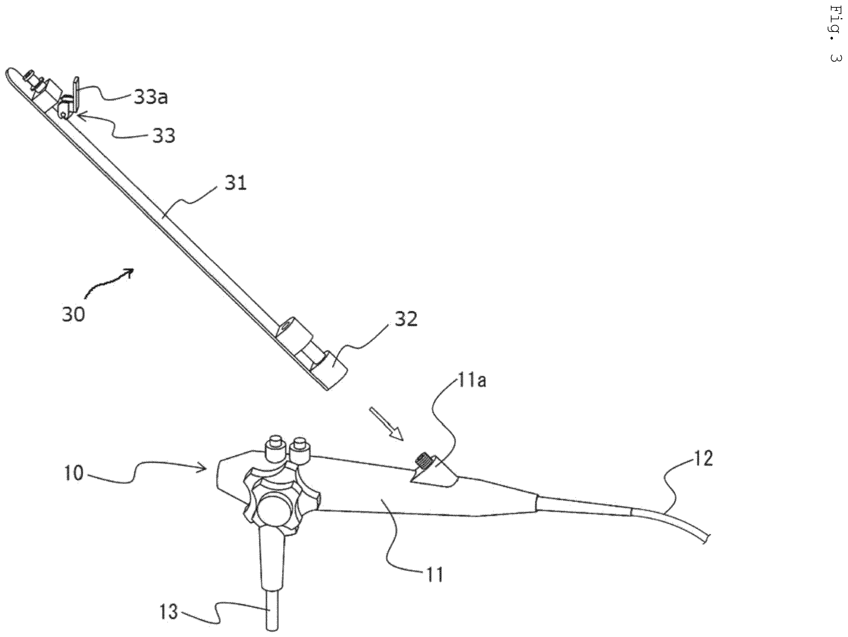

First, as illustrated in FIG. 3, the connector portion 32 provided at one end of the rod 31 of the assisting device 30 is fixed and mounted on the forceps insertion port 11a of the endoscope 10. With this, the assisting device 30 is removably fixed to the endoscope 10. Next, the outer tube 22 having the inner tube 21 inserted therein is inserted from the forceps insertion port 11a of the endoscope 10 and caused to pass through the conduit in the endoscope 10. Then, the distal end of the inner tube 21 and the distal end of the outer tube 22 are caused to protrude from the forceps port 11b at the distal end of the insertion tube 12 of the endoscope 10, and the water-absorbing material 23 is also caused to protrude. In this case, the branch connector 24 of the outer tube 22 is positioned between the connector portion 32 and the fixture 33.

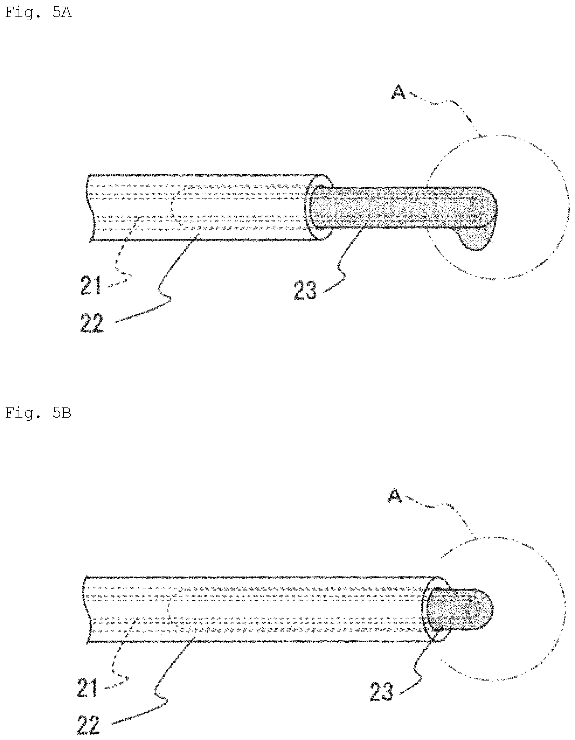

Then, the inner tube 21 extending from the branch connector 24 to the proximal end side is inserted in the fixture 33 of the assisting device 30. In this state, the position of the water-absorbing material 23 protruding from the distal end forceps port 11b at the distal end of the insertion tube 12 of the endoscope 10 is adjusted to confirm that the inner tube 21 and the outer tube 22 are not loosened, and the inner tube 21 is fixed by turning the lock lever 33a of the fixture 33 to the lock position. In this case, the distal end of the inner tube 21 is usually arranged in an affected area A (FIG. 5A) that is a target or a site for applying the liquid drug under the endoscope 10, or in the vicinity the affected area A. The affected area A may be a tumor or a site that causes a disease. The outer tube 22 and the inner tube 21 positioned between the connector portion 32 and the fixture 33 of the assisting device 30 are in parallel to the rod 31. Examples of the tumor include, but not limited to, stomach cancer, colorectal cancer, esophagus cancer, and bladder cancer. Examples of the "disease" in the "site that causes a disease" include intractable gastrointestinal discomfort, intractable airway hyperresponsiveness, intractable bladder discomfort, interstitial cystitis, intractable frequent urination, and pelvic organ pain, which are diseases except the tumor.

The endoscope 10 is a ready-made product. Therefore, the distance from the forceps insertion port 11a to the forceps port at the distal end of the insertion tube 12 is constant, and hence the fixing position of the inner tube 21 with the fixture 33 can be specified in advance. In view of the foregoing, the above-mentioned fixing operation can also be simplified by forming marking at the position specified in advance and performing the operation through use of the marking as a mark.

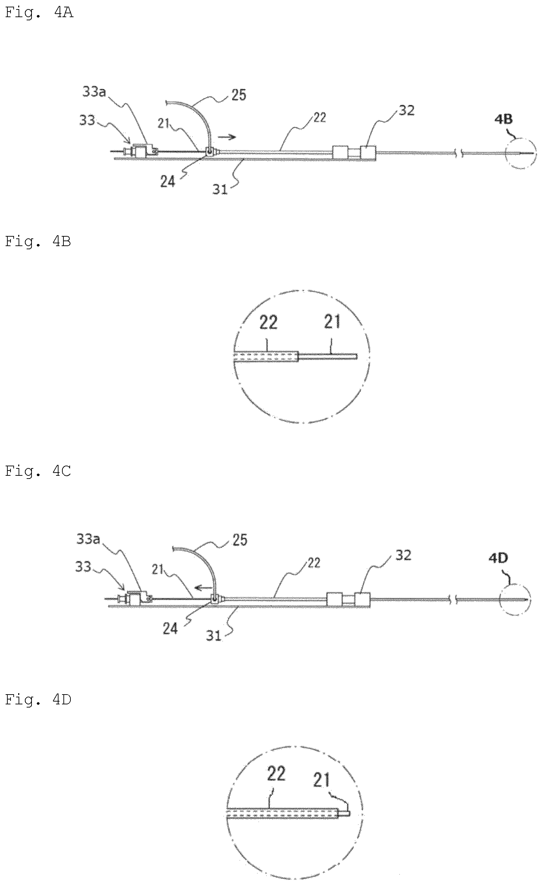

As described above, the inner tube 21 is fixed with the fixture 33, and the outer tube 22 is allowed to selectively advance and retract with respect to the inner tube 21. Therefore, as illustrated in FIG. 4A to FIG. 4D, only the outer tube 22 can be operated so as to selectively advance and retract with respect to the inner tube 21.

In particular, the outer tube 22 is in parallel to the rod 31 between the connector portion 32 and the fixture 33. Therefore, when the operator, such as a physician, holds the outer tube 22 of a portion between the connector portion 32 and the branch connector 24 with the hand through use of the rod 31 as a guide to advance the outer tube 22, the smooth advancing operation from the state illustrated in FIG. 4A to the state illustrated in FIG. 4C can be performed.

The liquid drug is supplied from the supply syringe 28 to the inner tube 21, and the water-absorbing material 23 mounted on the distal end of the inner tube 21 is impregnated with the liquid drug. As illustrated in FIG. 5A, in the case where the liquid accumulation occurs when permeation treatment of applying the liquid drug to the affected area A through use of the water-absorbing material 23 is performed, the liquid accumulation can be eliminated by advancing the outer tube 22 to retract a part of the water-absorbing material 23 into the outer tube 22 as illustrated in FIG. 5B. By moving the distal end of the outer tube 22 to a target position where the liquid is to be aspirated, the liquid drug can be accurately aspirated. In addition, when the water-absorbing material 23 is pulled into the outer tube 22, the water-absorbing material 23 more strongly receives the aspiration action of the outer tube 22 in a negative pressure state, and the liquid accumulation can be eliminated in a shorter period of time, preferably instantaneously.

After the liquid accumulation is eliminated, the outer tube 22 is retracted from the state illustrated in FIG. 4C to the state illustrated in FIG. 4A to be restored to the state before the advancing operation. In this state, the permeation treatment of the liquid drug to the affected area with the water-absorbing material 23 can be resumed.

The effects of the liquid drug permeation apparatus and the liquid drug permeation system according to the first embodiment of the present invention are described.

In the liquid drug permeation apparatus 20 and the liquid drug permeation system 100 according to the first embodiment of the present invention, the inner tube 21 is coaxially inserted in the inside of the outer tube 22, and a part of the liquid drug supplied through the inner tube 21 is aspirated through the outer tube 22. In addition, the water-absorbing material 23 mounted on the distal end of the inner tube 21 is wetted with the liquid drug, and the water-absorbing material 23 is brought into contact with a predetermined affected area, to thereby cause the liquid drug to permeate to the affected area.

In particular, the distal end of the inner tube 21 is caused to protrude as compared to the distal end of the outer tube 22, and the outer tube 22 is allowed to selectively advance and retract with respect to the inner tube 21. Therefore, when the liquid accumulation leading to the liquid dripping of the liquid drug occurs in the affected area A, the outer tube 22 is advanced with respect to the inner tube 21 at suitable timing by the operator to retract a part of the water-absorbing material 23 into the outer tube 22. By increasing a region of the water-absorbing material 23 subjected to the action of aspiration of the liquid drug by the outer tube 22, and also by bringing the distal end of the outer tube 22 close to the liquid accumulation in a site for aspiration to enhance the aspiration force to the site, the liquid accumulation is eliminated to eliminate the risk of the liquid dripping.

By moving the distal end of the outer tube 22 to a target position where the liquid is to be aspirated, the liquid drug can be accurately aspirated.

When the water-absorbing material 23 is pulled into the outer tube 22, the water-absorbing material 23 more strongly receives the aspiration action of the outer tube 22 in a negative pressure state, and the liquid accumulation can be eliminated in a shorter period of time, preferably instantaneously.

The liquid accumulation can be eliminated without changing the supply flow rate of the liquid drug from the inner tube 21 and the aspiration pressure of the liquid drug by the outer tube 22. Therefore, the state before the occurrence of the liquid accumulation can be restored only by returning the outer tube 22 from the advanced position to the position before the advancing operation. Therefore, the permeation operation of the liquid drug by the liquid drug permeation apparatus 20 and the liquid drug permeation system 100 can be continuously performed, and the operation efficiency can also be improved.

Previously, in a state without elimination means for the liquid accumulation of the present invention, in order to suppress the occurrence of the liquid accumulation, the permeation treatment has hitherto been performed under a state in which the supply flow rate of the liquid drug from the inner tube 21 is reduced. However, in the liquid drug permeation apparatus 20 and the liquid drug permeation system 100 according to this embodiment, the occurrence of the liquid dripping can be prevented even when the supply flow rate of the liquid drug from the inner tube 21 is increased, and hence the operability of the permeation treatment can also be improved.

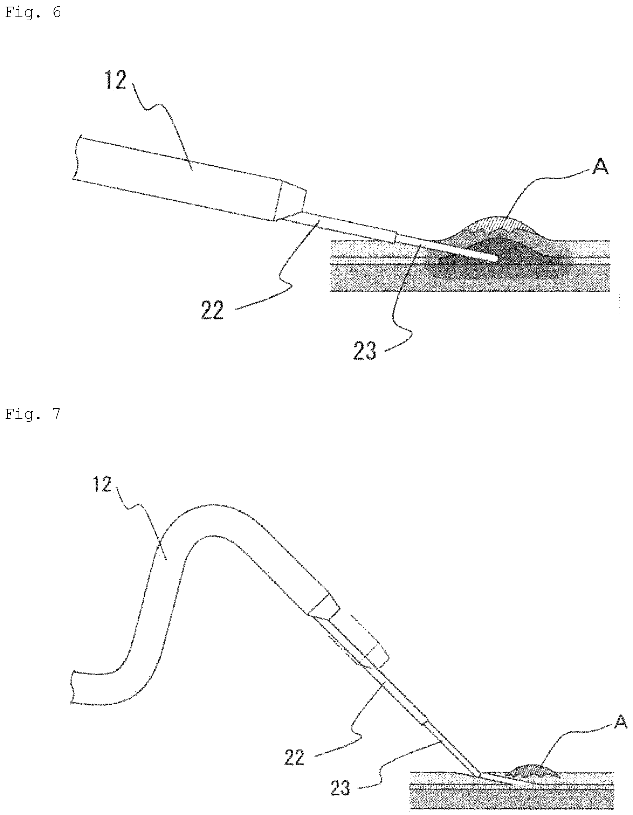

Instead of the use mode of applying the liquid drug to the surface of an affected area, the liquid drug may be filled into the affected area or caused to permeate thereto by inserting the inner tube 21 in the affected area as illustrated in FIG. 6. In the case of such a use mode, when the inner tube 21 is not fixed, the inner tube 21 is not successfully inserted in the affected area in some cases.

Specifically, the tissue of an affected area, such as cancer, is hard in many cases. Therefore, when the outer tube 22 is advanced together with the inner tube 21 in order to insert the inner tube 21 in the affected area, the inner tube 21 is pushed into the outer tube 22 due to the resistance from the tissue, and the inner tube 21 cannot be inserted in the tissue as intended. In addition, as illustrated in FIG. 7, the force for inserting the inner tube 21 in the affected area is replaced by a force for deforming the outer tube 22, with the result that the inner tube 21 is not successfully inserted in the affected area in some cases.

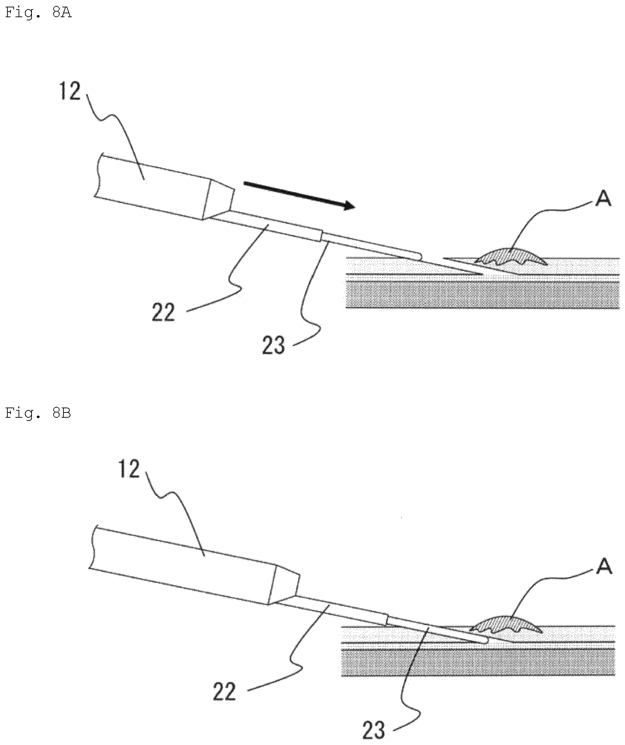

In order to insert the inner tube 21 in the tissue, it is required that the inner tube 21 be fixed to the assisting device 30 and that the inner tube 21 be advanced together with the insertion tube 12 of the endoscope 10 as illustrated in FIG. 8A and FIG. 8B. When the outer tube 22 is brought into a state of being guided by the insertion tube 12, the deformation of the outer tube 22 is suppressed, and the inner tube 21 can be reliably inserted in the affected area with a stronger force.

The liquid drug permeation apparatus and the liquid drug permeation system according to the first embodiment of the present invention can be effectively used for prevention or a therapy of a tumor, or discomfort alleviation, pain relief, and ablation therapies in sites such as the peripheral nerve causing other diseases.

The first embodiment of the present invention has been described above. However, the present invention is not limited thereto, and various modifications can be made as described below.

In the above-mentioned embodiment, as illustrated in FIG. 3, the connector portion 32 is provided at one end portion of the rod 31, and the fixture 33 is provided at another end portion of the rod 31. However, the positions of the connector portion 32 and the fixture 33 are not limited to the end portions of the rod 31. It is only required that the connector portion 32 and the fixture 33 be provided at different places of the rod 31.

In the embodiment illustrated in FIG. 3, the assisting device 30 is removably fixed to the endoscope 10. However, the assisting device 30 may be built in the endoscope 10 in advance, and the assisting device 30 may be irremovably fixed to the endoscope 10.

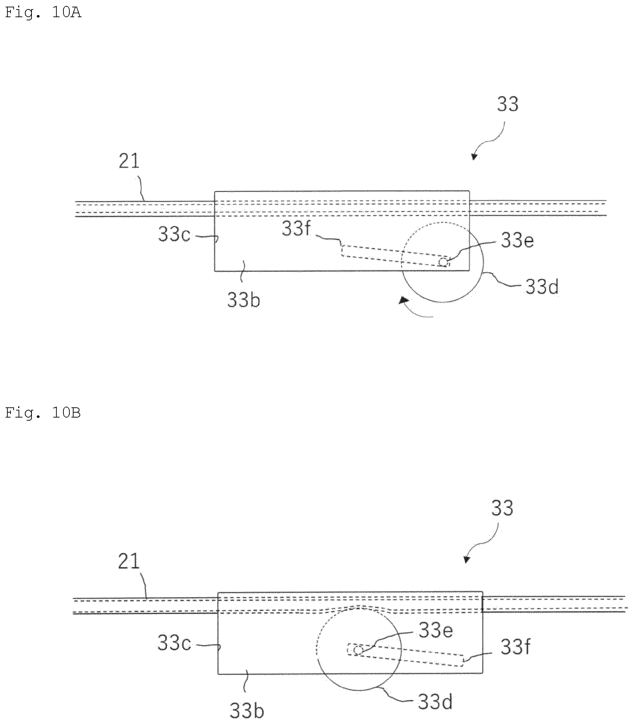

In the above-mentioned embodiment, the position of the inner tube 21 with respect to the assisting device 30 is fixed with the fixture 33 including the wedge plate (not shown) configured to crush the rubber tube toward the center and the lock lever 33a connected to the wedge plate. However, any suitable fixture configured to fix the position of the inner tube 21 with respect to the assisting device 30 by applying an external pressure to the inner tube 21 may be used. For example, the fixture 33 according to such an embodiment is illustrated in FIG. 10A and FIG. 10B. The fixture 33 includes a main body 33b having a substantially U-shape in cross-section to be fixed to the assisting device 30, and the inner tube 21 is inserted in the main body 33b through an opening portion 33c defined by the main body 33b. That is, the fixture 33 is mounted on the inner tube 21 at a place where only the inner tube 21 is exposed without the outer tube 22. A wall facing the opening portion 33c has a long groove 33f for accommodating a rod 33e configured to pivotally support a wheel 33d having a substantially circular shape. The groove 33f is formed such that, as compared to one end of the groove 33f close to an end portion in a longitudinal direction of the main body 33b, another end of the groove 33f close to a center in the longitudinal direction of the main body 33b extends so as to be separated from an end portion in a transverse direction (direction perpendicular to the longitudinal direction) of the main body 33b close to the one end of the groove 33f and is inclined with respect to the longitudinal direction of the main body 33b. In a release state illustrated in FIG. 10A, the rod 33e is located at a distance from the inner tube 21, and hence the wheel 33d is released without restricting the movement of the inner tube 21, and the inner tube 21 can move along the longitudinal direction thereof through the opening portion 33c. Then, when the operator rotates the wheel 33d with the hand in the direction indicated by the arrow, the rod 33e moves in the groove 33f and approaches the inner tube 21. As illustrated in FIG. 10B, when the wheel 33d is brought into contact with the inner tube 21 due to the movement along the groove 33f of the rod 33e and further presses the inner tube 21 to move, the inner tube 21 is brought into contact with a wall defining the opening portion 33c of the main body 33b, and the inner tube 21 is fixed between the wall defining the opening portion 33c of the main body 33b and the wheel 33d. That is, the movement of the inner tube 21 is restricted. With such a fixture, the position of the inner tube 21 with respect to the assisting device 30 can be fixed. Even in a state in which the position of the inner tube 21 is fixed, a lumen of the inner tube 21 is not completely closed. Although the inner tube 21 is slightly crushed by the wheel 33d in FIG. 10B, when the inner tube 21 is made of a hard material, the inner tube 21 may be fixed between the wall defining the opening portion 33c of the main body 33b and the wheel 33d without being deformed by the wheel 33d.

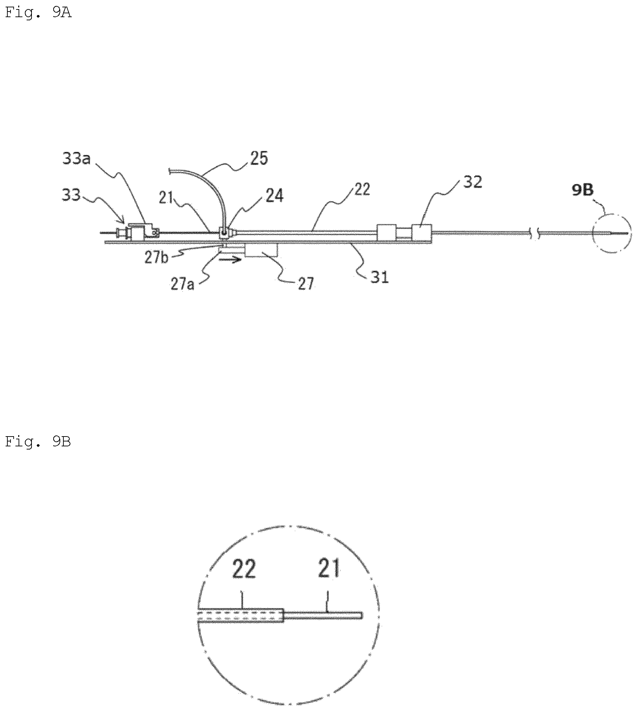

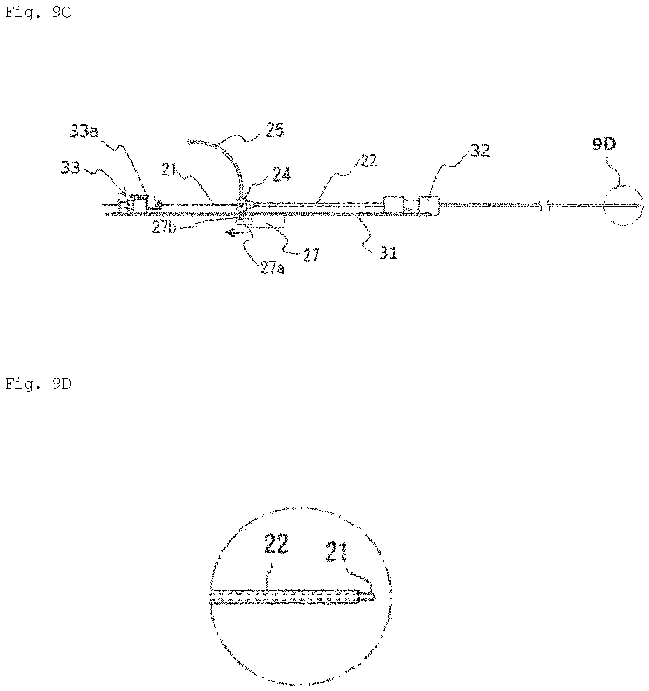

As illustrated in FIG. 9A to FIG. 9D, instead of allowing the operator to hold the outer tube 22 with the hand and advance the outer tube 22, for example, an outer tube movement drive device 27 configured to advance or retract the outer tube 22 by a predetermined distance may be provided in the middle of the rod 31. The advancing and retracting operation by the outer tube movement drive device 27 can be achieved through use of an appropriate actuator. For example, in the outer tube movement drive device 27 illustrated in FIG. 9A to FIG. 9D, an advancing and retracting rod 27a configured to selectively advance and retract along the extending direction of the rod 31 and the branch connector 24 are coupled to each other through a coupling arm 27b, and the branch connector 24 is advanced and retracted by the advancing and retracting operation of the advancing and retracting rod 27a to selectively advance and retract the outer tube 22.

Further, the outer tube movement drive device 27 may be connected to a computer 35 including a storage unit and a processor. By performing the following setting: when a predetermined signal is input to the computer 35, the computer 35 sends, to the outer tube movement drive device 27, a command of causing the outer tube movement drive device 27 to automatically advance the outer tube 22 by a predetermined distance based on the signal and then retract the outer tube 22, an excess liquid drug can be aspirated at appropriate timing, and the liquid accumulation can be more reliably eliminated.

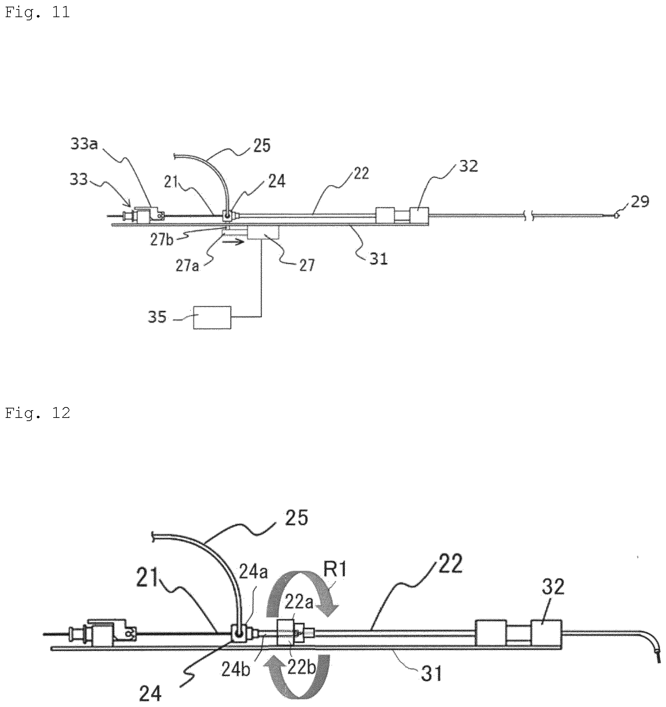

Such a predetermined signal may be: data input by the manual operation of the computer, utterance, or pressing of switches with the hand and foot of the operator, such as a physician; data stored in the storage unit of the computer 35 and read therefrom; data indicating the state of the periphery of the affected area A acquired from a sensing means 29 (see FIG. 11), such as a contact/tactile switch, a contact/tactile sensor, or an image sensor; data, such as image data, position data, and gravity direction data, on the periphery of the affected area A acquired from the endoscope 10; and/or data acquired by further analyzing, with analysis means, the data indicating the state of the periphery of the affected area A acquired from the sensing means 29 or the endoscope 10. Any one of those data is sent to the computer 35.

When such a signal is input, the computer 35 determines whether or not a) the movement of the outer tube, b) the movement of the inner tube, c) the aspiration of the liquid drug through the outer tube, or d) the supply of the liquid drug through the inner tube is required, and controls so as to perform or so as not to perform any one of a) to d) based on the determination result. The criterion of the determination may be a reference value determined by the operator, such as a physician, from the degree of the liquid accumulation on the periphery of the affected area A or the like, and other well-known reference values indicating the state of the affected area A may be used.

For example, regarding a) and b), the distance of movement, the speed of movement, the strength of contact when the distal end of each tube is brought into contact with a target tissue, and the like can be automatically controlled by the computer 35. Regarding c) and d), the strength or change rate of aspiration of the liquid drug, the intensity or change rate of supply (injection) of the liquid drug, the frequency of aspiration or supply of the liquid drug, and the like can be automatically controlled by the computer 35.

In particular, when the sensing means 29 and the computer 35 are used in combination, in order to check the operation situation with the endoscope, as illustrated in FIG. 11, the sensing means 29, such as the contact/tactile switch, the contact/tactile sensor, or the image sensor, is provided at the distal end of the inner tube 21 or the distal end of the outer tube 22, and the sensing means 29 acquires the data on the periphery of the affected area A. Next, the computer 35 that is communicably connected to the sensing means 29 receives the data acquired by the sensing means 29, and/or the data, such as the image data, the position data, and the gravity direction data, on the periphery of the affected area A acquired by the endoscope 10. The computer 35, artificial intelligence (AI) provided in another device, or another analysis means analyzes the data, automatically detects the occurrence of the liquid accumulation on the periphery of the affected area A, and sends a predetermined signal related to the occurrence of the liquid accumulation to the computer 35. The computer 35 having received the predetermined signal related to the occurrence of the liquid accumulation sends a command to the outer tube movement drive device 27 so that the outer tube movement drive device 27 performs the advancing operation of the outer tube 22. Then, the outer tube movement drive device 27 advances the outer tube 22. Thus, the detection of the occurrence of the liquid accumulation and the aspiration of an excess liquid drug can be automatically performed. The computer 35, the artificial intelligence (AI), or another analysis means function also as a automatic liquid supply control device.

The automation of the operation of each component of the liquid drug permeation apparatus assisting device 30 and the liquid drug permeation apparatus 20 under the endoscope can be performed, for example, by linking the automation of advancing and retracting of an aspiration tube (outer tube 22) and the automation of aspiration of an excess drug to each other, performing the following programing in the computer 35, and repeatedly performing a series of operations (1) to (6) in the following order.
(1) Regarding [b: Movement of Inner Tube]: In this program, the relative positional relationship between the endoscope 10 and the injection tube (inner tube 21) is temporarily fixed by the assisting device 30, to thereby prevent the inner tube 21 from moving.
(2) Regarding [d: Supply of Liquid Drug through Inner Tube]: 50% acetic acid (50% of the balance is an indigocarmine undiluted solution) is always injected continuously at 40 µL/min.
(3) The computer 35 receives the above-mentioned predetermined signal or a predetermined signal related to the above-mentioned occurrence of the liquid accumulation.
(4) Regarding [a: Movement of Outer Tube]: Only the aspiration tube (outer tube 22) is slightly advanced so as to cover a distal end portion of the injection tube (inner tube 21) (the aspiration force can be increased by advancing). In addition, the liquid dripping can be more reliably avoided by bringing the distal end of the aspiration tube into direct contact with a portion in which the liquid dripping is liable to occur to aspirate an excess drug.
(5) Regarding [c: Aspiration of Liquid Drug through Outer Tube]: An excess drug, such as the liquid accumulation, is aspirated intermittently and periodically once every 30 seconds at a speed of 1.5 mL/sec. The operation of aspiration is performed in synchronization with the operation from the start of advancing of the outer tube 22 to the end of retracting of the outer tube 22.
(6) Regarding [a: Movement of Outer Tube]: The aspiration tube (outer tube 22) is retracted by the same distance through which the aspiration tube has been advanced, to thereby restore the outer tube 22 to a state before the outer tube 22 is advanced. By the time when this restoration is completed, (5) [c: Aspiration of Liquid Drug through Outer Tube] is finished.

Further, the following method is conceivable. When the liquid drug is applied to a target site or an affected area with the water-absorbing material 23 while the distal end of the endoscope 10 is shaken horizontally and vertically, the computer 35 receives the data acquired from the sensing means 29 and the data, such as image data, position data, and gravity direction data, on the periphery of the site acquired from the endoscope 10. Then, the computer 35 brings the distal end of the injection tube (inner tube 21) into contact with the target site to apply the liquid drug thereto while bending the injection tube (inner tube 21) itself or the distal end of the aspiration tube (outer tube 22) itself horizontally and vertically through use of automatic control or AI control. For example, as a material for the inner tube 21 for that purpose, a commercially available distal end movable therapeutic microcatheter "Leonis Mova (trademark)" (manufactured by Sumitomo Bakelite Co., Ltd.) may be used.

Further, the following method is conceivable. When the liquid drug is applied to a target site under the endoscope, for example, a site to which the liquid drug is applied is irradiated with a laser pointer or the like mounted on the endoscope 10, and the computer 35 receives the irradiation site together with the data acquired from the sensing means 29 and the data, such as image data, position data, and gravity direction data, on the periphery of the site acquired from the endoscope 10. Then, the computer 35 guides the distal end of the injection tube (inner tube 21) or the aspiration tube (outer tube 22) to the irradiation site through use of automatic control or AI control and brings the distal end of the injection tube (inner tube 21) into contact with the target site to apply the liquid drug thereto.

AI is, in particular, machine learning, and in order for a machine to learn, data to be a basis for learning (for example, the data on the periphery of the affected area A from the sensing means 29 and/or the data, such as image data, position data, and gravity direction data, on the periphery of the affected area A acquired from the endoscope 10) is used as an input value. The input value is processed by a machine learning algorithm to find out a process for classifying and recognizing the data. Through use of the learned process, even data that has been input after learning and has not been learned can also be classified and identified. Through machine learning, classification, recognition, identification, or prediction can be performed.

In the liquid drug permeation systems according to the first embodiment (see FIG. 4A and FIG. 4C), the second embodiment (see FIG. 9A and FIG. 9C), and the embodiment illustrated in FIG. 11, the branch connector 24 is mounted on the proximal end portion of the outer tube 22, and one end of the coupling tube 25 is connected to the side surface of the branch connector 24. However, the proximal end portion of the outer tube 22 and the branch connector 24 having one end of the coupling tube 25 connected thereto may be separable. Specifically, as illustrated in FIG. 12, the branch connector 24 having one end of the coupling tube 25 connected thereto includes a hollow shaft portion 24b in fluid communication to the annular main body 24a of the branch connector 24. An annular base connector 22a is mounted on the proximal end portion of the outer tube 22, and the shaft portion 24b of the branch connector 24 is separably mounted on a lumen of the base connector 22a. In order to facilitate or support rotation of the outer tube 22 by the operator, the outer diameter of an annular main body 22b of the base connector 22a is formed to be larger than the outer diameter of the outer tube 22. When the base connector 22a is rotated in a direction indicated by an arrow R1, the branch connector 24 is arranged at a position separated from the base connector 22a or in a state of enabling rotation of the base connector 22a. When the piston of the aspiration syringe 26 (FIG. 2) is aspirated to aspirate the liquid drug in the outer tube 22 through the coupling tube 25, the branch connector 24 is connected to the base connector 22a so as to be in fluid communication to the outer tube 22.

In the first embodiment (see FIG. 4A and FIG. 4C), the second embodiment (see FIG. 9A and FIG. 9C), and the embodiment illustrated in FIG. 11, when the distal end of the inner tube 21 and the distal end of the outer tube 22 are simultaneously rotated, the branch connector 24 having the inner tube 21 inserted therein is rotated. In this case, depending on the degree of rotation of the branch connector 24, the coupling tube 25 connected to the branch connector 24 may bump into the rod 31 to restrict the rotation, or the coupling tube 25 may move against the intention of the operator due to the influence of the gravity on the coupling tube 25, with the result that a suitable rotation state of the branch connector 24 is not kept.

However, in this example, when the operator intends to rotate each of the distal ends of the inner tube 21 and the outer tube 22, the base connector 22a mounted on the proximal end portion of the outer tube 22 is rotated in the direction indicated by the arrow R1, and torque (torsional strength) can be technically easily generated in the outer tube 22 irrespective of the presence of the coupling tube 25. As a result, the operator can easily achieve the rotation of the base connector 22a and the rotation of each of the distal ends of the inner tube 21 and the outer tube 22 based on the rotation of the base connector 22a. In addition, the operator can easily maintain the rotation state of the base connector 22a and the rotation state of each of the distal ends of the inner tube 21 and the outer tube 22.

Further, the endoscope described herein encompasses a hard endoscope as well as a soft endoscope. In addition, the endoscope described herein encompasses an industrial endoscope as well as a medical endoscope.

### Examples

### Example 1

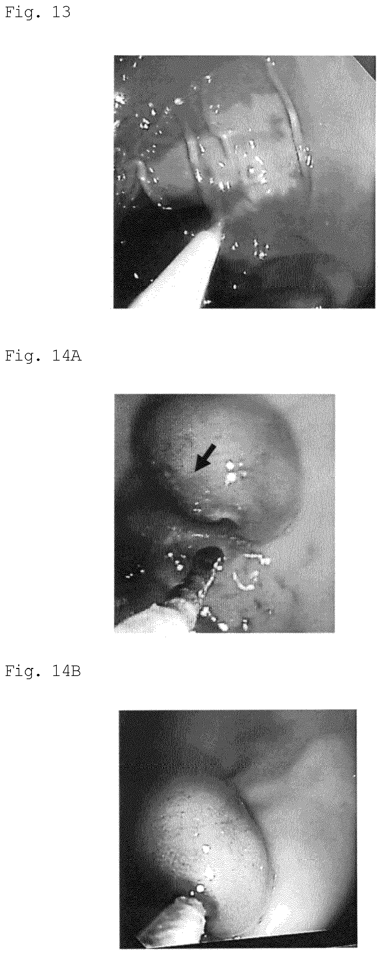

1. An upper gastrointestinal soft endoscope was inserted in the stomach of a beagle dog (adult dog, male) under general anesthesia. After that, an endoscopic liquid drug permeation apparatus assisting device and an endoscopic liquid drug permeation apparatus (hereinafter referred to as "assisting device" and "liquid drug permeation apparatus") corresponding to the endoscopic liquid drug permeation apparatus assisting device 30 and the endoscopic liquid drug permeation apparatus 20 thereof illustrated in FIG. 1A were mounted on the endoscope.
2. In an injection tube (inner tube), 50 vol% acetic acid (glacial acetic acid was diluted with pure water) was always injected continuously at 40 µL/min. With this, the 50% (vol%, the same applies hereinafter) acetic acid was supplied to a distal end of the injection tube in the figure, and the 50% acetic acid was applied to the stomach wall mucosa (normal stomach wall mucosa in this case) assumed to be a therapeutic target. This application action was performed and accomplished by allowing the operator to grip the upper gastrointestinal soft endoscope with both hands and slightly moving the upper gastrointestinal soft endoscope to bring the distal end of the injection tube into light contact with the stomach wall mucosa while observing a monitor image of the endoscope.
3. Aspiration from an aspiration tube (outer tube) was manually performed through use of a syringe for 3 seconds intermittently and periodically once every 30 seconds at a speed of 1.5 mL/sec, and this aspiration was repeated. When this aspiration was performed, the operator performed an operation involving, in order to quickly aspirate the 50% acetic acid that was liable to drip with an increased aspiration force, slightly advancing only the aspiration tube so as to cover the distal end portion of the injection tube without moving the position of the injection tube in the assisting device and the liquid drug permeation apparatus.
   When the operation of advancing only the aspiration tube was performed, the relative positional relationship between the injection tube and the endoscope main body was fixed by the assisting device, and hence the relative positional relationship between the aspiration tube and the injection tube when the aspiration tube is moved forward and backward was able to be controlled suitably and accurately. After the aspiration, only the aspiration tube was retracted in the same manner as in the operation of advancing only the aspiration tube in advance, and the aspiration tube was restored to a state before being advanced.
4. Air in the gastric cavity was sucked in association with the aspiration in the above-mentioned section 3, and the air in the gastric cavity was being reduced. Therefore, in order to avoid the reduction in air, air in the same amount as that aspirated in the section 3 was sent into the gastric cavity from a forceps channel of the endoscope in synchronization with the aspiration in the above-mentioned section 3. That is, air was manually sent through use of a syringe for 3 seconds intermittently and periodically once every 30 seconds at a speed of 1.5 mL/sec, and thus, the air was repeatedly sent. With this, the amount of a gas, such as air, in the gastric cavity having the endoscope distal end inserted therein was kept substantially constant, and artificial nmovement and displacement of the stomach wall caused by treatment or the like were suppressed.
5. As a result of the treatment in the above-mentioned section 4, maintenance of the cavity pressure and maintenance and securing of a viewing field were enabled, and accurate application of the 50% acetic acid was able to be performed together with the effect of the treatment in the above-mentioned section 3. Drips and the like were accurately and quickly aspirated in order to avoid the liquid dripping to prevent the liquid dripping of the 50% acetic acid, and the 50% acetic acid was able to be prevented from adhering to a place other than a target site.
6. A photograph of the stomach wall mucosa after about 0.5 mL of the 50% acetic acid was applied in the performance of the above-mentioned sections 1 to 5 is shown in FIG. 13. The site having the 50% acetic acid applied thereto became white as compared to the other sites.

### Example 2

1. An upper gastrointestinal soft endoscope was inserted in the stomach of a beagle dog (adult dog, male) under general anesthesia. An endoscope puncture needle (manufactured by Top Corporation) was inserted in a treatment tool channel (forceps insertion port 11a of FIG. 1A) of the endoscope, and a needle of a distal end portion of the endoscope puncture needle was inserted in the normal stomach wall mucosa. Then, the mucosa portion was artificially raised by injecting physiological saline, and a pseudo raised portion was formed in a tumor. After that, the endoscope puncture needle was removed from the treatment tool channel, and a disposable high-frequency knife (manufactured by Olympus Corporation) serving as an endoscope treatment tool was inserted to coagulate a part of the raised portion, to thereby form a small hole (portion indicated by the solid arrow in FIG. 14A). After that, the disposable high-frequency knife was removed from the treatment tool channel, and an endoscopic liquid drug permeation apparatus assisting device and an endoscopic liquid drug permeation apparatus (hereinafter referred to as "assisting device" and "liquid drug permeation apparatus") corresponding to the endoscopic liquid drug permeation apparatus assisting device 30 and the endoscopic liquid drug permeation apparatus 20 thereof illustrated in FIG. 1A were mounted on the endoscope.
2. The operator gripped the upper gastrointestinal soft endoscope with both hands and integrally advanced the upper gastrointestinal soft endoscope and the "endoscopic permeation apparatus" while observing a monitor image of the endoscope, to thereby insert a distal end of an injection tube (inner tube) in the stomach wall mucosa through the small hole. When an operation of integrally advancing the upper gastrointestinal soft endoscope and the "endoscopic permeation apparatus" was performed, the relative positional relationship between the injection tube and the endoscope main body was fixed by the "endoscopic permeation apparatus assisting device". Therefore, a portion covered with cotton of the distal end portion of the injection tube was able to be inserted in the mucosa (stomach wall tissue) and advanced while the injection tube, an aspiration tube, or the endoscope main body were prevented from warping (being bent). As shown in FIG. 14B, when the relative positional relationship between the endoscope and the injection tube (inner tube) was temporarily fixed by the assisting device, the distal end portion of the injection tube was able to be inserted in the mucosa (stomach wall tissue) and advanced by the operation of physically pushing the endoscope and the injection tube under a state in which the endoscope and the injection tube were integrated.

### Reference Signs List

10 endoscope
11a forceps insertion port
11b distal end forceps port
20 liquid drug permeation apparatus
21 inner tube
22 outer tube
23 water-absorbing material
26 aspiration syringe forming aspiration device
27 outer tube movement drive device
28 supply syringe forming liquid drug supply device
29 sensing means
30 assisting device
31 rod serving as elongated body
32 connector portion
33 fixture
35 computer functions also as automatic liquid supply control device
100 liquid drug permeation system
A affected area that is target or site for applying liquid drug

## Claims

1. An assisting device (30) for a liquid drug permeation apparatus (20) under an endoscope (10),
wherein the liquid drug permeation apparatus (20) includes an outer tube (22) and an inner tube (21) inserted in the outer tube (22), the inner tube (21) has a water-absorbing material (23) for absorbing a liquid drug mounted on a distal end, and a part of the liquid drug supplied through the inner tube (21) is aspirated through the outer tube (22),
wherein the assisting device (30) includes:
a connector portion (32) to be mounted on the endoscope (10); and
a fixture (33) configured to fix the inner tube (21) to the assisting device (30), and
wherein, when the connector portion (32) of the assisting device (30) is mounted on the endoscope (10) and the inner tube (21) is fixed to the assisting device (30) with the fixture (33), the outer tube (22) is allowed to selectively advance and retract with respect to the inner tube (21), and the water-absorbing material (23) is allowed to be pulled into the outer tube (22) by advancing the outer tube (22) with respect the inner tube (21).

2. The assisting device (30) according to claim 1, wherein
the assisting device (30) includes an elongated body,
the elongated body includes the connector portion (32) and the fixture (33), and
when the inner tube (21) is fixed to the assisting device (30) with the fixture (33), the outer tube (22) and the elongated body are made parallel.

3. The assisting device (30) according to claim 1 or 2, further comprising an outer tube movement drive device (27) configured to advance the outer tube (22) by a predetermined distance with respect to the inner tube (21).

4. The assisting device (30) according to any one of claims 1 to 3, further comprising a base connector (22a), which is to be mounted on a proximal end portion of the outer tube (22), and which is configured to support rotation of the outer tube (22).

5. An endoscope (10) comprising the assisting device (30) of any one of claims 1 to 4 irremovably fixed thereto.

6. A liquid drug permeation system (100) for causing permeation of a liquid drug under an endoscope (10), comprising:
a liquid drug permeation apparatus (20); and
an assisting device (30) for the liquid drug permeation apparatus (20) under the endoscope (10) according to any one of claims 1 to 4.

7. The liquid drug permeation system (100) according to claim 6, wherein
the liquid drug permeation system (100) further comprises the endoscope (10) including a forceps insertion port (11a) and a distal end forceps port (11b),
the connector portion (32) of the assisting device (30) is mounted on the forceps insertion port (11a),
the inner tube (21) and the outer tube (22) of the liquid drug permeation apparatus (20) are inserted in the forceps insertion port (11a) of the endoscope (10) and are caused to extend from the distal end forceps port (11b),
the water-absorbing material (23) is allowed to protrude from the distal end forceps port (11b), and
when the inner tube (21) is fixed with the fixture (33), the outer tube (22) is allowed to advance with respect to the inner tube (21).

8. The liquid drug permeation system (100) according to claim 7, wherein the assisting device (30) is irremovably fixed to the endoscope (10).

9. The liquid drug permeation system (100) according to any one of claims 6 to 8, wherein
the inner tube (21) of the liquid drug permeation apparatus (20) is connected to a liquid drug supply device (28), and the liquid drug is supplied from the liquid drug supply device (28) to the inner tube (21), and
the outer tube (22) of the liquid drug permeation apparatus (20) is connected to an aspiration device (26), and the liquid drug is aspirated into the outer tube (22) by operation of the aspiration device (26).

10. The liquid drug permeation system (100) according to any one of claims 6 to 9, wherein a sensing means (29) for sensing a state of an affected area to which the liquid drug is caused to permeate or a state of a periphery of the affected area is provided at the distal end of the inner tube (21) or a distal end of the outer tube (22).

11. The liquid drug permeation system (100) according to claim 10, further comprising a automatic liquid supply control device, which is communicably connected to the sensing means (29), and which is configured to control movement of the outer tube (22), movement of the inner tube (21), aspiration of the liquid drug through the outer tube (22), or supply of the liquid drug through the inner tube (21), based on data obtained from the sensing means (29) on the affected area to which the liquid drug is caused to permeate or the periphery of the affected area.

## Patentansprüche

1. Hilfsvorrichtung (30) für eine Vorrichtung (20) zur Permeation flüssiger Medikamente unter einem Endoskop (10),
wobei die Vorrichtung (20) zur Permeation flüssiger Medikamente ein äußeres Rohr (22) und ein in das äußere Rohr (22) eingesetztes inneres Rohr (21) umfasst, das innere Rohr (21) ein an einem distalen Ende angebrachtes wasserabsorbierendes Material (23) zum Absorbieren eines flüssigen Medikaments aufweist und ein Teil des durch das innere Rohr (21) zugeführten flüssigen Medikaments durch das äußere Rohr (22) abgesaugt wird,
wobei die Hilfsvorrichtung (30) umfasst:
einen Verbindungsabschnitt (32) zum Anbringen an dem Endoskop (10); und
eine Befestigungsvorrichtung (33), die konfiguriert ist zum Befestigen des inneren Rohrs (21) an der Hilfsvorrichtung (30), und
wobei, wenn der Verbindungsabschnitt (32) der Hilfsvorrichtung (30) an dem Endoskop (10) angebracht ist und das innere Rohr (21) mit der Befestigungsvorrichtung (33) an der Hilfsvorrichtung (30) befestigt ist, das äußere Rohr (22) in Bezug auf das innere Rohr (21) selektiv vorgeschoben und zurückgezogen werden kann und das wasserabsorbierende Material (23) in das äußere Rohr (22) gezogen werden kann, indem das äußere Rohr (22) in Bezug auf das innere Rohr (21) vorgeschoben wird.

2. Hilfsvorrichtung (30) nach Anspruch 1, wobei
die Hilfsvorrichtung (30) einen länglichen Körper aufweist,
der längliche Körper den Verbindungsabschnitt (32) und die Befestigungsvorrichtung (33) umfasst und
wenn das innere Rohr (21) mit der Befestigungsvorrichtung (33) an der Hilfsvorrichtung (30) befestigt wird, das äußere Rohr (22) und der längliche Körper parallel werden.

3. Hilfsvorrichtung (30) nach Anspruch 1 oder 2, die ferner eine Antriebsvorrichtung (27) zum Bewegen des äußeren Rohrs umfasst, die konfiguriert ist zum Vorschieben des äußeren Rohrs (22) um eine vorbestimmte Strecke in Bezug auf das innere Rohr (21).

4. Hilfsvorrichtung (30) nach einem der Ansprüche 1 bis 3, ferner umfassend ein Basisverbindungsstück (22a), das an einem proximalen Endabschnitt des äußeren Rohrs (22) anzubringen ist und das zur Unterstützung einer Drehung des äußeren Rohrs (22) konfiguriert ist.

5. Endoskop (10), das die Hilfsvorrichtung (30) gemäß einem der Ansprüche 1 bis 4 umfasst, die unbeweglich daran befestigt ist.

6. System (100) zur Permeation flüssiger Medikamente zum Bewirken einer Permeation eines flüssigen Medikaments unter einem Endoskop (10), umfassend:
eine Vorrichtung (20) zur Permeation flüssiger Medikamente; und
eine Hilfsvorrichtung (30) für die Vorrichtung (20) zur Permeation flüssiger Medikamente unter dem Endoskop (10) gemäß einem der Ansprüche 1 bis 4.

7. System (100) zur Permeation flüssiger Medikamente nach Anspruch 6, wobei
das System (100) zur Permeation flüssiger Medikamente ferner das Endoskop (10) umfasst, das eine Zangeneinführöffnung (11a) und am distalen Ende eine Zangenöffnung (11b) aufweist,
der Verbindungsabschnitt (32) der Hilfsvorrichtung (30) an der Zangeneinführöffnung (11a) angebracht ist,
das innere Rohr (21) und das äußere Rohr (22) der Vorrichtung (20) zur Permeation flüssiger Medikamente in die Zangeneinführöffnung (11a) des Endoskops (10) eingeführt werden und dazu gebracht werden, sich von der Zangenöffnung (11b) am distalen Ende aus zu erstrecken,
das wasserabsorbierende Material (23) aus der Zangenöffnung (11b) am distalen Ende herausragen kann und
wenn das innere Rohr (21) mit der Befestigungsvorrichtung (33) fixiert ist, das äußere Rohr (22) in Bezug auf das innere Rohr (21) vorgeschoben werden kann.

8. System (100) zur Permeation flüssiger Medikamente nach Anspruch 7, wobei die Hilfsvorrichtung (30) am Endoskop (10) unbeweglich befestigt ist.

9. System (100) zur Permeation flüssiger Medikamente nach einem der Ansprüche 6 bis 8, wobei
das innere Rohr (21) der Vorrichtung (20) zur Permeation flüssiger Medikamente mit einer Zuführvorrichtung (28) für flüssige Medikamente verbunden ist und das flüssige Medikament von der Zuführvorrichtung (28) für flüssige Medikamente dem inneren Rohr (21) zugeführt wird, und
das äußere Rohr (22) der Vorrichtung (20) zur Permeation flüssiger Medikamente mit einer Absaugvorrichtung (26) verbunden ist und das flüssige Medikament durch Betätigen der Absaugvorrichtung (26) in das äußere Rohr (22) abgesaugt wird.

10. System (100) zur Permeation flüssiger Medikamente nach einem der Ansprüche 6 bis 9, wobei am distalen Ende des inneren Rohrs (21) oder einem distalen Ende des äußeren Rohrs (22) eine Erfassungseinrichtung (29) zum Erfassen eines Zustands eines betroffenen Bereichs, zu den das flüssige Arzneimittel permeiert werden soll, oder eines Zustands einem Umfeld des betroffenen Bereichs vorgesehen ist.

11. System (100) zur Permeation flüssiger Medikamente nach Anspruch 10, das ferner eine automatische Steuereinrichtung für die Flüssigkeitszufuhr umfasst, die mit der Erfassungseinrichtung (29) kommunizierend verbunden ist und die konfiguriert ist zur Steuerung der Bewegung des äußeren Rohrs (22), der Bewegung des inneren Rohrs (21), dem Absaugen des flüssigen Medikaments durch das äußere Rohr (22) oder der Zufuhr des flüssigen Medikaments durch das innere Rohr (21) basierend auf von der Erfassungseinrichtung (29) erhaltener Daten über den betroffenen Bereich, zu den das flüssige Arzneimittel permeiert werden soll, oder das Umfeld des betroffenen Bereichs.

## Revendications

1. Dispositif d'assistance (30) pour un appareil d'infiltration de médicament liquide (20) sous un endoscope (10),
dans lequel l'appareil d'infiltration de médicament liquide (20) inclut un tube externe (22) et un tube interne (21) inséré dans le tube externe (22), le tube interne (21) présente un matériau hydro-absorbant (23) pour absorber un médicament liquide monté sur une extrémité distale, et une partie du médicament liquide fourni à travers le tube interne (21) est aspirée à travers le tube externe (22),
dans lequel le dispositif d'assistance (30) inclut :
une portion de raccord (32) à monter sur l'endoscope (10) ; et
un élément de fixation (33) configuré pour fixer le tube interne (21) au dispositif d'assistance (30), et
dans lequel, lorsque la portion de raccord (32) du dispositif d'assistance (30) est montée sur l'endoscope (10) et que le tube interne (21) est fixé au dispositif d'assistance (30) avec l'élément de fixation (33), le tube externe (22) peut avancer et reculer sélectivement par rapport au tube interne (21), et le matériau hydro-absorbant (23) peut être tiré dans le tube externe (22) en avançant le tube externe (22) par rapport au tube interne (21).

2. Dispositif d'assistance (30) selon la revendication 1, dans lequel
le dispositif d'assistance (30) inclut un corps allongé,
le corps allongé inclut la portion de raccord (32) et l'élément de fixation (33), et
lorsque le tube interne (21) est fixé au dispositif d'assistance (30) avec l'élément de fixation (33), le tube externe (22) et le corps allongé sont rendus parallèles.

3. Dispositif d'assistance (30) selon la revendication 1 ou 2, comprenant en outre un dispositif d'entraînement de déplacement de tube externe (27) configuré pour avancer le tube externe (22) d'une distance prédéterminée par rapport au tube interne (21).

4. Dispositif d'assistance (30) selon l'une quelconque des revendications 1 à 3, comprenant en outre un raccord de base (22a), qui est à monter sur une portion d'extrémité proximale du tube externe (22), et qui est configuré pour permettre la rotation du tube externe (22).

5. Endoscope (10) comprenant le dispositif d'assistance (30) selon l'une quelconque des revendications 1 à 4 fixé à demeure à celui-ci.

6. Système d'infiltration de médicament liquide (100) pour provoquer l'infiltration d'un médicament liquide sous un endoscope (10), comprenant :
un appareil d'infiltration de médicament liquide (20) ; et
un dispositif d'assistance (30) pour l'appareil d'infiltration de médicament liquide (20) sous l'endoscope (10) selon l'une quelconque des revendications 1 à 4.

7. Système d'infiltration de médicament liquide (100) selon la revendication 6, dans lequel
le système d'infiltration de médicament liquide (100) comprend en outre l'endoscope (10) incluant un orifice d'insertion de pince (11a) et un orifice pour pince d'extrémité distale (11b),
la portion de raccord (32) du dispositif d'assistance (30) est montée sur l'orifice d'insertion de pince (11a),
le tube interne (21) et le tube externe (22) de l'appareil d'infiltration de médicament liquide (20) sont insérés dans l'orifice d'insertion de pince (11a) de l'endoscope (10) et sont amenés à s'étendre depuis l'orifice pour pince d'extrémité distale (11b),
le matériau hydro-absorbant (23) peut faire saillie depuis l'orifice pour pince d'extrémité distale (11b), et
lorsque le tube interne (21) est fixé avec l'élément de fixation (33), le tube externe (22) peut avancer par rapport au tube interne (21).

8. Système d'infiltration de médicament liquide (100) selon la revendication 7, dans lequel le dispositif d'assistance (30) est fixé à demeure à l'endoscope (10).

9. Système d'infiltration de médicament liquide (100) selon l'une quelconque des revendications 6 à 8, dans lequel
le tube interne (21) de l'appareil d'infiltration de médicament liquide (20) est raccordé à un dispositif d'alimentation en médicament liquide (28), et le médicament liquide est fourni du dispositif d'alimentation en médicament liquide (28) au tube interne (21), et
le tube externe (22) de l'appareil d'infiltration de médicament liquide (20) est raccordé à un dispositif d'aspiration (26), et le médicament liquide est aspiré dans le tube externe (22) par actionnement du dispositif d'aspiration (26).

10. Système d'infiltration de médicament liquide (100) selon l'une quelconque des revendications 6 à 9, dans lequel un moyen de détection (29) pour détecter un état d'une zone affectée dans laquelle le médicament liquide est amené à s'infiltrer ou un état d'une périphérie de la zone affectée est prévu au niveau de l'extrémité distale du tube interne (21) ou d'une extrémité distale du tube externe (22).

11. Système d'infiltration de médicament liquide (100) selon la revendication 10, comprenant en outre un dispositif de commande d'alimentation en liquide automatique, qui est raccordé en communication au moyen de détection (29), et qui est configuré pour commander le déplacement du tube externe (22), le déplacement du tube interne (21), l'aspiration du médicament liquide à travers le tube externe (22), ou l'alimentation en médicament liquide à travers le tube interne (21), sur la base de données obtenues à partir du moyen de détection (29) concernant la zone affectée dans laquelle le médicament liquide est amené à s'infiltrer ou la périphérie de la zone affectée.
